(19) Europäisches Patentamt
European Patent Office
Office européen des brevets

(11) **EP 2 025 744 A1**

(12) **DEMANDE DE BREVET EUROPEEN**

(43) Date de publication:
**18.02.2009 Bulletin 2009/08**

(51) Int Cl.:
*C12M 1/34* (2006.01)     *G01N 33/487* (2006.01)

(21) Numéro de dépôt: **08158182.9**

(22) Date de dépôt: **13.06.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **15.06.2007  FR 0704302**

(71) Demandeur: **Nanotec Solution
30900 Nîmes (FR)**

(72) Inventeurs:
• **Esteban, Geoffrey
  30900 Nimes (FR)**
• **Luong, Bruno
  30900 Nimes (FR)**
• **Ossart, Frédéric
  30980 Langlade (FR)**

(74) Mandataire: **Pontet, Bernard
  Pontet Allano & Associés s.e.l.a.r.l.
  25, Rue Jean Rostand
  Parc Club Orsay Université
  91893 Orsay Cedex (FR)**

(54) **Procédé et système pour compter en ligne et in situ des cellules dans un milieu de culture biologique**

(57)     Procédé pour compter en ligne et *in situ* dans cellules dans un milieu de culture biologique, comprenant les étapes suivantes:
-une pluralité de mesures de la capacitance du milieu ou une pluralité de mesures de la conductance de ce milieu, à des fréquences distinctes variant dans une plage de fréquences de mesure prédéterminée,
- une extraction d'une information de variation de permittivité due à la β-dispersion dans ce milieu, à partir des mesures de capacitance, et
- un traitement de la variation de cette information de variation de permittivité en fonction de la fréquence, pour délivrer une information de comptage de cellules dans ledit milieu.

FIG.1

EP 2 025 744 A1

**Description**

**1 Introduction**

La présente invention concerne un procédé pour compter en ligne et *in situ* des cellules dans un milieu de culture biologique. Elle vise également un système de comptage de cellules mettant en oeuvre ce procédé.

Les industries de la pharmacie et de la biotechnologie utilisent des cellules animales recombinées pour produire des molécules d'intérêts thérapeutiques en bioréacteur. Parmi les mesures disponibles pour l'optimisation et à la bonne conduite de la réaction, la quantité de biomasse est un paramètre d'intérêt primordial. La mesure de la biomasse est aujourd'hui indispensable pour caractériser les procédés de culture biologique, ce paramètre biomasse est mesuré lors de toutes les cultures de cellules.

La mesure de référence est le comptage du nombre de cellules par microscopie après coloration au bleu trypan. Les cellules sont préalablement mis en contact avec un colorant (bleu trypan), et seules les cellules qui laissent pénétrer le colorant sont considérées mortes. Ainsi il est possible de compter les cellules vivantes (non colorées), les cellules mortes (colorés au bleu trypan), le nombre total et d'en déduire le pourcentage de cellules vivantes par rapport au nombre total de cellules, ou viabilité.

De nombreux fournisseurs proposent cette méthode et des microscopes associés à un traitement d'image pour effectuer ces différents comptages. Ces solutions sont plus ou moins automatisées mais la mesure se fait avec un échantillonnage du réacteur.

D'autres méthodes hors ligne (avec prise d'échantillon) sont utilisées en complément de cette référence comme le PCV (packed cell volume), le comptage par « coulter counter », le comptage par cytométrie de flux, la mesure du poids sec, l'analyse de la quantité d'ADN...

En culture biologique, la mesure en ligne et *in situ* des paramètres apportent un avantage très important par rapport à la mesure d'un échantillon: mesure en temps réel, automatisation de la mesure, possibilité d'utiliser la mesure en boucle de régulation, pas de risque de contamination lié à l'échantillonnage.

Ainsi de nombreux fournisseurs proposent des capteurs de biomasse en ligne et in situ pour la culture biologique. Ces capteurs de biomasse mettent en oeuvre des méthodes optiques ou capacitives.

Les méthodes optiques comprennent des mesures d'absorbance ou d'atténuation de la lumière plus communément appelée turbidité. La turbidité du milieu de culture est corrélée avec la quantité de biomasse en suspension. Le signal optique mesuré est corrélé à la quantité de particules en suspension ou le nombre de cellules totales (vivante et morte).

Certains capteurs de biomasse mettent en oeuvre des mesures de rétrodiffusion dans lesquelles le signal optique mesuré est corrélé à la quantité de particules en suspension et à leur taille. Ce signal donne une information sur la quantité de biomasse totale (vivante et morte).

Sont également utilisées des techniques de microscopie *in situ,* dans lesquelles toutes les cellules (vivantes et mortes) sont directement comptées et le nombre de cellules totales est accessible.

Parmi les techniques de mesure en ligne, c'est la technique de mesure capacitive qui est la méthode la plus performante et attractive car le signal obtenu est en relation avec la biomasse vivante uniquement. En effet seules les cellules vivantes sont actives, se divisent, ou produisent les molécules thérapeutiques ciblées, ce sont celles -ci que l'on souhaite mesurer en premier. Cette technique donne des mesures qui sont sensibles à la fois au nombre et la taille des cellules vivantes.

Cependant aucune mesure en ligne n'est capable de mesurer à la fois le nombre (indépendamment du changement de la taille des cellules) et à la fois la taille des cellules comme les mesures de référence hors ligne décrites ci-dessus.

**2 Objet de l'invention**

L'objet de l'invention est de proposer un procédé de comptage qui serait mis en oeuvre dans un capteur, en ligne et in situ, de mesure de biomasse en culture biologique, ce capteur étant capable de mesurer le nombre de cellules vivantes et leur taille, le nombre de cellules totale, et le pourcentage de cellules vivantes par rapport au nombre total de cellules, ou viabilité.

**3 Etat de l'art**

Pour un exposé de la théorie de base sur les mesures capacitives sur des cultures biologiques, on pourra se référer utilement aux travaux de (Pethig and Kell 1987; Foster and Schwan 1989; Markx and Davey 1999).

Il y est notamment fait référence à la relation liant permittivité et fréquence d'excitation, dans une mesure de permittivité d'un milieu de cellules en suspension.

La mesure de capacitance dans un milieu de culture de cellules, consiste en premier lieu à appliquer une pluralité de fréquences d'excitation comprises entre 0.1 et 20 MHZ, et de mesurer la permittivité à chaque fréquence. Le spectre obtenu contient trois types d'informations qui se superposent :

- La permittivité due à la β-dispersion des cellules
- La permittivité due à la polarisation des électrodes
- La permittivité parasite de l'électronique

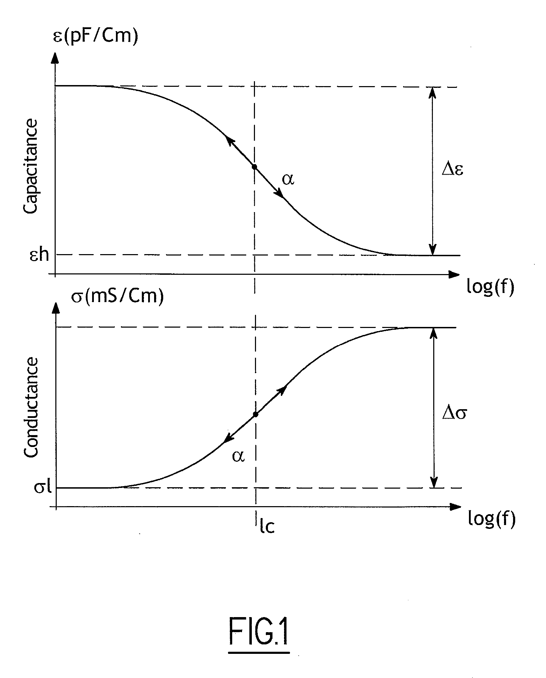

La chute de permittivité, causée par l'accumulation d'une double couche d'ions à proximité de la surface des électrodes, est appelée la polarisation des électrodes. La chute de la permittivité est causée par la polarisation des membranes cellulaires. Cette chute de la permittivité est appelée β-dispersion, en référence à la figure 1.
La β-dispersion peut elle-même être décrite par trois paramètres :

- l'amplitude de la β-dispersion, $\Delta\varepsilon$
- la fréquence caractéristique fc,
- le coefficient $\alpha$ de Cole-Cole.

Il est bien connu pour un homme de l'art que la permittivité due à la β-dispersion contient les informations des cellules en suspension comme : le nombre de cellules, le rayon moyen, la capacité membranaire Cm des cellules, et la conductivité du cytoplasme.
Pour extraire ces informations des cellules à partir des mesures capacitances, la méthode la plus classique consiste à les traiter en deux étapes :

- Correction : enlever la polarisation de l'électrode et la permittivité parasite de l'électronique dans le spectre de la permittivité mesuré pour obtenir le spectre de la β-dispersion.
- Extraction du rayon et du nombre de cellules à partir de la β-dispersion corrigée.

Plusieurs variantes de méthodes existent pour ces deux étapes.

Pour la première étape :

- La correction de la polarisation basée sur la mesure de la permittivité dans la région à basse fréquence où la contribution de la β-dispersion dans la permittivité totale est faible (typiquement <500 kHz). [Cannizzaro 2003, Asami 1995]. Ces méthodes sont basées sur l'hypothèse que la β-dispersion est constante en basse fréquences. Pour les cellules de large taille, cette hypothèse n'est plus valide et cette méthode introduit des erreurs de correction.
- Bordi [Bordi 2001] a proposé une méthode globale qui détermine la contribution de la polarisation des électrodes de type : : $C_p = Pxf^{pp}$, où P est l'amplitude de la polarisation, *f* est la fréquence, et $1 < pp \leq 2$. Cette méthode ne repose sur aucune hypothèse sur la forme de la β-dispersion par rapport à la plage de fréquence mesurée, et elle permet de déterminer à la fois *P* et *pp* par un fit (ajustement) non linéaire sur un circuit électrique équivalent. Le modèle utilisé par Bordi est un modèle complexe qui englobe les trois contributions dans la mesure de la permittivité :

- la contribution due à la β-dispersion des cellules
- la contribution due à la polarisation des électrodes et
- la contribution due aux parasites de l'électronique.

Cette approche requiert la disponibilité simultanément de la partie réelle (conductance) et complexe (capacitance) de la permittivité mesurée. La gamme de fréquence utilisée par Bordi est extrêmement large (de quelque kHz à plusieurs centaines de MHz). Cette gamme de fréquence dépasse largement les gammes de fréquences utilisées couramment dans les applications de culture biologique industrielles. D'autre part, cette gamme nécessite l'utilisation d'un impédancemètre de laboratoire très haut de gamme dont le prix et le caractère non-industriel est rédhibitoire dans l'application de culture biologique industrielle.
Pour la deuxième étape, il existe trois approches :
Une première approche consiste à extraire le nombre de cellules et le rayon directement à partir du spectre de capacitance. Cette approche fait appel à des méthodes statistiques comme l'analyse des composantes principales (PCA), et la méthode des moindres carrés partiels (PLS) [Cannizzaro 2003].
Cette méthode est opérée directement sur le spectre de permittivité, corrigé ou brut, mais elle prend difficilement en compte le caractère fortement non-linéaire de la dépendance du rayon des cellules aux mesures capacitives. La mise en oeuvre de ces méthodes statistiques est lourde, car elle nécessite au préalable une étape de culture biologique dite de « calibration », où les mesures de capacitances seraient confrontées à une mesure hors ligne du nombre de cellules et leurs rayons.

Les cultures de calibration doivent être réalisées de telle sorte que toute la gamme de rayon de cellules et la gamme des nombres de cellules susceptible d'être mesurés par la suite soient totalement couvertes par la culture de calibration. Cette calibration est spécifique à un type de cellule, et à une condition de culture spécifique. Dès que l'une de ces deux facteurs change, une nouvelle calibration est nécessaire. Cela est une difficulté majeure afin de généraliser la méthode et la rendre pratique dans un objectif applicatif industrielle.

Une telle méthode, qui implique autant de calibrations que de type de cellule, est difficilement implémentable en tant que procédé industriel de comptage en ligne.

Une deuxième approche consiste à extraire les paramètres par fitting (ajustement): Un modèle empirique de type Cole/Cole est utilisé pour décrire la β-dispersion. Ensuite, les trois paramètres décrivant la β-dispersion, $\Delta\varepsilon$, fc, $\alpha$ sont extraites par un fit (ajustement) non-linéaire. Le fit (ajustement) est opéré sur les données après la correction de la polarisation de type basse-fréquence [Yardley & al 2000]. L'implémentation du fit (ajustement) est une étape particulièrement critique, car les paramètres estimés sont imprécis à cause du caractère non-linéaire et instable du modèle par rapport à des paramètres Cole/Cole. Généralement le fit (ajustement) est réalisé par des algorithmes de type génétique, des réseaux de neurones [Nicholson 1996], des procédés de type « simulation annealing » [Lising 1996], ou Levenberg-Marquardt [Davey 1992 , Davey 1993].

Du fait de ces enchaînements séquentiels, l'erreur de la correction de polarisation induit une erreur des paramètres $\Delta\varepsilon$, fc et $\alpha$, surtout lorsque des cellules de grande taille sont mesurées. Aucune de ces méthodes n'est totalement fiable pour être applicable à un large éventail de cultures biologiques.

Ces paramètres pourraient être ensuite utilisés pour estimer le nombre et la taille des cellules par des calculs. Les calculs sont basés sur un modèle de dispersion des cellules sphériques appelé Pauly-Schwan que nous détaillons dans la suite. Le plus souvent, la capacité membranaire Cm des cellules, et la conductivité du cytoplasme sont les paramètres qui doivent être connus pour pouvoir estimer la taille et le nombre des cellules. Ces paramètres pourraient être estimés par des mesures hors ligne du type électrorotation [Archner, 1999].

Une troisième approche a été proposée par [Asami 1995], qui met en oeuvre une formule algébrique permettant d'estimer la fréquence caractéristique fc avec la mesure de conductance et de capacitance à une fréquence d'excitation prédéfinie. Les inconvénients de cette méthode sont que cette détermination est dépendante la fréquence appliqué (dépendance d'ordre 2) ce qui nécessite la calibration en fréquence de l'impédancemètre utilisé et la fréquence appliquée doit être proche de fc selon les recommandations D'Asimi.

D'autre part, cette méthode demande une calibration du début de la culture (t=0). Ce qui suppose que la capacité membranaire et la conductance intracellulaire ne varient pas au cours de la fermentation.

## 4 Exposé de l'invention

Le but de l'invention est de remédier aux inconvénients posés par les procédés de comptage de l'art antérieur, tant en termes d'exploitabilité industrielle que de prise en compte des erreurs de polarisation.

Cet objectif est atteint avec un procédé pour compter en ligne et *in situ* des cellules dans un milieu de culture biologique, comprenant les étapes suivantes:

- une pluralité de mesures de la capacitance dudit milieu ou une pluralité de mesure de la conductance dudit milieu, à des fréquences distinctes variant dans une plage de fréquences de mesure prédéterminée,
- une extraction d'une information de variation de permittivité due à la β-dispersion dans ledit milieu, à partir desdites mesures de capacitance, et
- un traitement de ladite information de variation de permittivité pour délivrer une information de comptage de cellules dans ledit milieu.

Ce procédé est basé sur une méthode permettant l'estimation de la taille moyenne des cellules et le dénombrement des cellules par des mesures diélectriques et conductances avec plusieurs fréquences.

Contrairement à des méthodes statistiques, le procédé de comptage selon l'invention revient à combiner de manière inattendue le modèle empirique de type Cole/Cole et un modèle décrivant la dispersion d'une cellule sphérique de type Pauly-Schwan.

L'avantage principal de cette invention réside dans sa précision et sa fiabilité de mesure avec tous types de cellules biologiques et dans toutes conditions environnementales de mesure. Contrairement à Bordi, les erreurs systématiques de l'électronique et de la polarisation font l'objet d'un prétraitement, ce qui simplifie ensuite la résolution des paramètres de β-dispersion.

Dans une version avantageuse, le procédé de comptage selon l'invention comprend en outre une étape préalable de calibration d'une culture desdites cellules, dite culture de calibration, cette étape de calibration comportant :

- (i) au moins une pluralité de mesures de permittivité de ladite culture de calibration à des fréquences prédéterminées,
- (ii) un traitement desdites mesures de permittivité pour calculer des facteurs de détermination des informations de comptage.

Un autre avantage du procédé selon l'invention réside ainsi dans le fait que la méthode mise en oeuvre ne nécessite aucune connaissance préalable de la capacité membranaire Cm des cellules, et de sa conductivité du cytoplasme. En contre partie, pour chaque type de culture, elle fait appele à une procédure d'auto-calibration qui ne demande que quelques points spécifiques (un à trois dans une réalisation préférentielle décrite plus loin) de la culture de calibration. L'auto-calibration consiste à corréler ces points avec une mesure indépendante de la taille et des nombres des cellules.

Dans le cadre du procédé de comptage des cellules vivantes selon l'invention, on réalise une culture préalable - dite de calibration - dans laquelle sont mesurés plusieurs spectres (un à trois dans la réalisation préférentielle) de permittivité à une multitude de fréquences et l'absorbance pour déterminer :

- un facteur $k_1$ ou les constantes (a, b, c) qui permet de déterminer le rayon dans les cultures suivantes avec la mesure de la fréquence critique fc,
- un facteur $k_2$ ou les constantes (d, e, f) qui permet de déterminer le nombre de cellules dans les cultures suivantes à l'aide de la mesure de $\Delta\varepsilon$ ou $\Delta\sigma$ et de la fréquence critique fc,
- un facteur K' qui permet de déterminer le nombre de cellules totales dans les cultures suivantes à l'aide de la mesure d'absorbance.

Les mesures du nombre total de cellules et du nombre de cellules vivantes dans les cultures suivantes seront utilisées pour déterminer la viabilité cellulaire.

Les constantes $\overline{k}_1$, $\overline{k}_2$, ou a, b, c, d, e, f, correspondent à deux réalisations préférentielles décrites plus loin.

L'estimation du nombre et de la taille des cellules vivantes se fait en deux étapes :

1. Déterminer les paramètres de la β-dispersion : l'amplitude $\Delta\varepsilon$ de la dispersion des capacitances ou l'amplitude $\Delta\sigma$ de la dispersion des conductances ; la fréquence caractéristique fc, et optionnellement le coefficient α de Cole-Cole.
2. Estimation de la taille et du nombre des cellules vivantes à partir de ces paramètres.

Suivant un autre aspect de l'invention, il est proposé un système pour compter en ligne et *in situ* des cellules dans un milieu de culture biologique, mettant en oeuvre un procédé selon l'invention, ce système comprenant :

- des moyens pour mesurer la capacitance dudit milieu, à des fréquences distinctes variant dans une plage de fréquences de mesure prédéterminée,
- des moyens pour extraire une information de variation de permittivité due à la β-dispersion dans ledit milieu, à partir desdites mesures de capacitance, et
- des moyens pour traiter ladite information de variation de permittivité, pour délivrer une information de comptage de cellules dans ledit milieu.

Le système de comptage selon l'invention peut avantageusement comprendre en outre des moyens pour réaliser une calibration d'une culture desdites cellules, dite culture de calibration, comportant :

- (i) des moyens pour effectuer des mesures de permittivité de ladite culture de calibration à des fréquences prédéterminées, et
- (ii) des moyens pour traiter lesdites mesures de permittivité, agencés pour calculer des facteurs de détermination des informations de comptage.

## 5. Description détaillée de l'invention

D'autres avantages et caractéristiques de l'invention apparaîtront à l'examen de la description détaillée d'un mode de mise en oeuvre nullement limitatif, et des dessins annexés sur lesquels :

- la figure 1 illustre le phénomène connu de β-dispersion sous la forme d'une évolution de la permittivité et de conductivité en fonction de la fréquence ;

- La figure 2 est un schéma synoptique d'un système de comptage de cellules selon l'invention ;
- La figure 3 illustre des étapes du procédé de comptage de cellules selon l'invention ;
- La figure 4 illustre une évolution des nombres de cellules respectivement vivantes et totales dans un milieu et de la permittivité en fonction du temps ;
- la figure 5 illustre une évolution des nombres de cellules respectivement vivantes et totales dans un milieu et de la variation de permittivité en fonction du temps ;
- la figure 6 illustre une évolution des nombres respectifs de cellules vivantes et de cellules totales et de l'absorbance en fonction du temps ; et
- la figure 7 illustre une évolution du rayon moyen de cellules dans un milieu en fonction du temps.

On va maintenant décrire différentes phases de détermination de paramètres réalisées lors de l'implémentation du procédé de comptage selon l'invention.

5.1 Description de l'estimation du nombre des cellules vivantes à partir des paramètres Cole/Cole

Un nombre de cellules en augmentation ou une modification de la taille des cellules a une influence sur le biovolume et sur l'amplitude du signal de permittivité, selon la première équation de Pauly-Schwan :

$$\Delta\varepsilon = \frac{9 \times r \times P \times C_M}{4} \qquad (1)$$

avec

$\Delta\varepsilon$ : permittivité (F m$^{-1}$), amplitude de la $\beta$-dispersion
r: rayon d'une cellule (m)
P: fraction volumique des cellules (biovolume)

$$P = \frac{4}{3} \times \pi \times r^3 \times Nv \qquad (2)$$

Nv: densité de cellules vivantes (m$^{-3}$)

$C_M$: Capacitance membranaire par unité de surface $\left(\dfrac{F}{m^2}\right)$

Il en résulte qu'un rayon de cellule r plus élevé a pour effet d'accroître l'amplitude $\Delta\varepsilon$ lorsque la fraction volumique P (biovolume) reste constante.
La fréquence caractéristique fc est définie par la deuxième équation de Pauly-Schwan :

$$f_C = \frac{1}{2 \times \pi \times r \times C_M \times \left(\dfrac{1}{\sigma_c} + \dfrac{1}{2\sigma_m}\right)} \qquad (3)$$

avec $\sigma_c$: conductivité du cytoplasme $\left(\dfrac{mS}{cm}\right)$

$\sigma_m$ : conductivité du milieu $\left[\overline{\quad \text{cm} \quad}\right]$

Cette fréquence critique fc est par conséquent fonction de la taille (r) de la cellule, de l'état de la cellule et des propriétés de la membrane cellulaire $C_M$.

La β-dispersion est une somme de l'ensemble des petites β-dispersions générées par chaque cellule distincte. Le paramètre Cole-Cole $\alpha$ représente la distribution des petites β-dispersions à la fréquence critique fc.

Les deux équations de Pauly-Schwan décrivent les deux paramètres de la β-dispersion en fonction des quatre paramètres cellulaires : $r$, $N_v$, $\sigma_M$, $\sigma_c$.

Dans les conditions de cultures biologiques rencontrées fréquemment, deux hypothèses peuvent êtres supposées :

- la capacitance membranaire Cm est une fonction connue de la température et de la conductivité du milieu de culture,
- La conductivité intracellulaire σi est une fonction connue de la température et de la conductivité du milieu de culture.

$$C_M = g(T, \sigma_m) \qquad (4)$$

$$\sigma_c = h(T, \sigma_m) \qquad (5)$$

Combinant ces deux équations (4) et (5) avec la deuxième équation de Pauly-Schwan (3), on obtient :

$$r = \frac{1}{2\pi} \times \frac{1}{g(T, \sigma_m)} \times \frac{2\sigma_m h(T, \sigma_m)}{2\sigma_m + h(T, \sigma_m)} \times \frac{1}{fc} \qquad (6)$$

Cette équation (6) peut être réécrite sous une forme simplifiée comme ceci :

$$r = k_1(T, \sigma_m) \times \frac{1}{fc} , \qquad (7)$$

avec :

$$k_1(T, \sigma_m) = \frac{1}{2\pi} \times \frac{1}{g(T, \sigma_m)} \times \frac{2\sigma_m h(T, \sigma_m)}{2\sigma_m + h(T, \sigma_m)} . \qquad (7.a)$$

Combinant la 1ere équation de Pauly-Schwan (1), l'équation de la fraction volumique (2) avec les deux équations (4) et (5), le nombre de cellules vivantes est donné par :

$$N_V = \frac{16\pi^3}{3} g(T, \sigma_m)^3 \left( \frac{2\sigma_m + h(T, \sigma_m)}{2\sigma_m h(T, \sigma_m)} \right)^4 \times \left( \Delta\varepsilon \times f_c^4 \right) \qquad (8)$$

Cette équation se réécrit sous une forme simplifiée :

$$N_V = k_2(T,\sigma_m) \times \left(\Delta\varepsilon \times f_c^4\right) \qquad (9)$$

avec :

$$k_2(T,\sigma_m) = \frac{16\pi^3}{3} g(T,\sigma_m)^3 \left(\frac{2\sigma_m + h(T,\sigma_m)}{2\sigma_m h(T,\sigma_m)}\right)^4. \qquad (9.a)$$

Le nombre de cellules vivantes est aussi exprimé en fonction de la différence de la conductance $\Delta\sigma$ et la fréquence fc. En effet, puisque $\Delta\sigma = 2\pi * fc * \Delta\varepsilon$, de (9), Nv est calculé d'une autre façon comme ceci :

$$N_V = \frac{1}{2\pi} \times k_2(T,\sigma_m) \times \left(\Delta\sigma \times f_c^3\right) \qquad (10).$$

En combinant les équations (7) et (9), ou (7) et (10), on établit que la détermination de $\Delta\varepsilon$ et de fc, ou de $\Delta\sigma$ et de fc permet une mesure directe et en ligne du nombre de cellules vivantes après détermination des fonctions $k_1$ et $k_2$ par une culture préalable et la corrélation avec une contre mesure du nombre de cellules vivantes, par exemple la méthode de référence citée ci-dessus.

Pour cela, on peut restreindre les fonctions $k1$ et $k2$ uniquement comme une somme pondérée des n fonctions de bases $\kappa_i$ :

$$k_1(T,\sigma_m) = \sum_{i=1,\dots n} c_i \times \kappa_i(T,\sigma) \qquad (11)$$

$$k_2(T,\sigma_m) = \sum_{i=1,\dots n} d_i \times \kappa_i(T,\sigma) \qquad (12).$$

Les coefficients $c_i$ et $d_i$ sont déterminés grâce à une culture de calibration et en faisant une corrélation des résultats donnés par (7) et (9) ou (7) et (10) avec au moins n résultats de mesure hors-ligne par une méthode de régression linéaire.

Une fois que la calibration est faite, c'est-à-dire une fois que les coefficients $c_i$ et $d_i$ sont déterminés, les tailles et le nombres des cellules vivantes pourraient être estimés en in-situ par les mesures capacitances, la température et la conductance du milieu avec les formules (7) (9) (11) (12), ou (7) (10) (11) (12).

Si la capacité membranaire Cm des cellules et la conductivité du cytoplasme $\sigma_c$ sont connues, les équations (7.a) et (9.a) sont utilisées à la place de (11) (12) pendant la mesure in-situ, et aucune culture de référence n'est nécessaire.

Le paramètre de Cole/Cole $\alpha$ fournit optionnellement une information qualitative sur la dispersion de la taille des cellules autour de sa valeur moyenne estimée par (7). Une formule empirique est employée permettant de relier $\alpha$ et le rayon moyen r et l'écart type de la dispersion de la taille.

On va maintenant décrire des modes préférentiels de réalisation pour cette étape de calibration.

1. Dans un premier mode de réalisation, on part du fait qu'il est démontré expérimentalement que pour la plupart des cultures, les deux fonctions $k_1$ et $k_2$ dépendent faiblement de la température et la conductivité du milieu. Ces deux fonctions pourraient être bien approximées par des fonctions linéaires de type :

$$k_1(T, \sigma_m) = a + b \times T + c \times \sigma_m$$

et

$$k_2(T, \sigma_m) = d + e \times T + f \times \sigma_m .$$

Les coefficients a, b, c, d, e, f sont déterminés avec au minimum trois expériences de la culture de référence dans le plan d'expérience ($T, \sigma_m$), et ensuite en faisant une corrélation avec une contre mesure du nombre de cellules vivantes, par exemple la méthode de référence citée ci-dessus.

2. Dans un second mode de réalisation correspondant à un calcul un peu moins précis des fonctions $k_1$ et $k_2$, mais beaucoup plus pratique à calibrer, c'est de les approximer par deux constantes $\overline{k}_1$ et $\overline{k}_2$ pour des applications de culture biologique dont la variation de température et de conductivité du milieu sont négligeables :

$$k_1(T, \sigma_m) = \overline{k}_1 \quad \text{et} \quad k_2(T, \sigma_m) = \overline{k}_2 .$$

Une seule mesure de la culture de référence suffit à caractériser les constantes $\overline{k}_1$ et $\overline{k}_2$, et en faisant une corrélation avec une contre mesure du nombre de cellules vivantes, par exemple la méthode de référence citée ci-dessus. La mesure de la température et de la conductance n'est pas nécessaire dans le cas de cette présente réalisation préférentielle.

5.2 Description de la détermination des paramètres de Cole/Cole à partir des spectres mesurés.

Deux méthodes alternatives sont proposées pour déterminer les paramètres de β-dispersion, nécessaires pour le comptage des cellules vivantes :

Détermination des paramètres $\alpha$,fC, $\Delta\epsilon$ par la méthode de fitting (ajustement)

Cette détermination est réalisée en deux sous étapes :

- Correction de la permittivité parasite de l'électronique et de la polarisation systématique des électrodes par soustraction ; Correction de la conductance parasite de l'électronique par soustraction selon procédé
- Correction de la permittivité due à la polarisation résiduelle des électrodes et détermination des paramètres Cole/Cole proprement dit par fitting (ajustement).
  Dans un premier temps, la permittivité et la conductance dues à l'électronique sont soustraites comme ceci :

$$\epsilon^{corr}(f, \sigma_m) = \epsilon^{mes}(f, \sigma_m) - \epsilon^{cal}(f, \sigma_m) \qquad (13)$$

$$\sigma^{corr}(f) = \sigma^{mes}(f) - \sigma^{cal}(f) \qquad (14).$$

Les permittivités $\epsilon^{cal}(f)$ et les conductances $\sigma^{cal}(f)$ sont mesurées par la sonde dans un milieu sans cellules, de même conductance $\sigma_m$ à basse fréquence que pendant la mesure de culture avec des cellules. Contrairement à [Bordi 2001]. Cette correction permet d'éliminer la contribution des capacitances parasites due à l'électronique dans la permittivité mesurée.

Les permittivités corrigées mesurées pour une pluralité de fréquences {f} seront ensuite fittées (ajustées) par un modèle de Cole/Cole, et augmentées par un terme de polarisation aléatoire :

$$\varepsilon(f) = \Delta\varepsilon \times \frac{1 + rf^{1-\alpha}\sin(\alpha\pi/2)}{1 + rf^{2(1-\alpha)} + 2 \times rf^{1-\alpha}\sin(\alpha\pi/2)} + \varepsilon_h + \Delta P \times f^{-pp}, \quad (15.a)$$

$$\sigma(f) = \Delta\sigma \times \frac{rf^{2-\alpha}\cos(\alpha\pi/2)}{1 + rf^{2(1-\alpha)} + 2 \times rf^{1-\alpha}\sin(\alpha\pi/2)} + \sigma_l, \quad (15.b)$$

avec

$$rf := \frac{f}{fc}.$$

Les cinq paramètres à déterminer sont :

$\alpha$ : paramètre de Cole/Cole,
$\Delta\varepsilon$ : amplitude de la dispersion de permittivité,
$f_c$ : fréquence caractéristique,
$\Delta P$ : amplitude de la polarisation résiduelle,
$\varepsilon_h$ : ligne de base, limite de la capacitance lorsque la fréquence
tend vers l'infini.

Les deux autres paramètres sont :

$\Delta_\sigma$ : amplitude de la dispersion de la conductance,
$\sigma_l$ : ligne de base, la conductance à basse fréquence

*pp* est une constante dite *puissance de polarisation.* La valeur de cette constante - comprise entre 1 et 2 - est propre à chaque sonde (notamment en fonction de sa géométrie et de la porosité de ses électrodes), et elle est caractérisée une seule fois pour la sonde.
Le calcul du fit (ajustement) est implémenté dans une unité de traitement en cherchant à minimiser une fonction de la distance « moindres - carrés » pondérée entre le modèle (15.a) et les données corrigées (13):

$$J(\alpha, \Delta\varepsilon, f_c, \Delta P, \varepsilon_h) = \sum_f w(f) \times \left(\varepsilon^{corr}(f) - \varepsilon(f)\right)^2 .$$

Les coefficients de pondération *w(f)* sont introduits pour compenser la différence de comportement du bruit électronique en fonction de la fréquence d'excitation.
Dans une implémentation préférentielle de la méthode, l'algorithme basé sur une variante d'un algorithme de type Quasi-Newton, Broyden-Fletcher-Goldfarb-Shanno (BFGS), à mémoire limitée avec préconditionnement, est utilisé pour minimiser J.
La variante introduite dans le cadre de la présente invention est une modification de l'algorithme BFGS classique afin de contraindre les paramètres dans leurs domaines de validité : $1 < \alpha \leq 2$, $\Delta\varepsilon \geq 0$, $f_c > 0$.
Suivant le type de culture biologique, d'autres contraintes portant sur les paramètres du modèle Cole/Cole pourraient être rajoutées dans l'algorithme afin de le rendre plus stable.
Dans une autre variante de la méthode, la partie réelle de la permittivité suivant le modèle de dispersion Cole/Cole (15.b) est combinée avec la partie imaginaire présentée dans (15.a) pour former un modèle de permittivité complexe utilisé par le fit (ajustement).
Ce modèle est fitté (ajusté) à la fois pour les mesures de la conductance et la capacitance corrigées (13) et (14). $\Delta\sigma$ et $\sigma_l$ s'ajoutent dans la liste des paramètres de fit (ajustement). Cette combinaison augmente la précision dans la détermination des paramètres de Cole/Cole.

Détermination des paramètres $\alpha$, fc, $\Delta\sigma$ par méthode algébrique

Les méthodes statistiques de type PLS ou par régression linéaire présentent les inconvénients (i) de nécessiter l'utilisation d'une large gamme de fréquences pour décrire la $\beta$-dispersion, et (ii) d'utiliser des algorithmes lourds. Cela rend le travail d'intégration dans des puces électroniques difficile.

La méthode algébrique a pour avantage d'être très facilement implantable dans un circuit intégré car elle est ultra légère en terme de calcul et en terme d'utilisation de fréquence. Elle n'utilise pas de régression linéaire et ne nécessite qu'un nombre minimal de fréquences de mesure.

Les paramètres $\alpha$, fc, $\Delta\sigma$ sont déterminés grâce à la résolution algébrique des modèles physiques de comportement de la cellule.

L'état de l'art nous incite à utiliser la spectroscopie de capacitance pour déterminer les paramètres caractéristiques $\alpha$, fc, $\Delta\varepsilon$. Or, la conductance est elle aussi affectée de cette dispersion.

La méthode consiste à combiner les modèles de $\beta$ dispersion de la conductance et de la capacitance afin de déterminer de façon algébrique les paramètres caractéristiques $\alpha$, fc, $\Delta\sigma$.

Cette méthode est utilisée de façon préférentielle à partir des expressions simplifiées de Pauly et Schwan lorsque $\alpha=0$:

$$\varepsilon(f) = \varepsilon_h + \frac{\Delta\varepsilon}{1 + \left(\dfrac{f}{fc}\right)^2} \qquad (16)$$

$$\sigma(f) = \sigma_l + \frac{\Delta\sigma\left(\dfrac{f}{fc}\right)^2}{1 + \left(\dfrac{f}{fc}\right)^2} \qquad (17)$$

$$\Delta\sigma = 2\pi * fc * \Delta\varepsilon \qquad (18)$$

La fréquence caractéristique fc est donc obtenue en combinant les expressions (16) (17) et (18)

$$fc = \frac{1}{2\pi} * \frac{\sigma(f1) - \sigma(f2)}{\varepsilon(f1) - \varepsilon(f2)} \qquad (19)$$

Avec seulement au moins deux fréquences, il est donc possible de déterminer la fréquence fc.

Le résultat de la combinaison des équations (16) (17) et (18) ne se limite pas seulement à cette forme, et elle peut être modifiée de sorte à utiliser plusieurs fréquences.

On peut également remarquer que contrairement à toutes les autres méthodes connues, cette détermination de fc est indépendante de tous les autres paramètres du milieu en suspension : du rayon r, de la capacité membranaire Cm, de la conductivité intra-cellulaire , du bio volume P, de la conductivité du milieu.

Cette détermination de la fréquence caractéristique fc est également indépendante des fréquences choisies. Il en résulte que la précision à laquelle sont appliquées les fréquences f1 et f2 importe peu, contrairement aux méthodes qui n'utilisent que la spectroscopie de capacitance. Ce qui offre l'avantage de ne pas nécessiter l'étalonnage en fréquence de l'impédancemètre utilisé.

Par ailleurs, la relation mathématique correspondante pour le calcul de la fréquence caractéristique fc est très facile à implémenter dans un circuit intégré.

On va maintenant décrire un mode pratique de détermination de la variation de conductance $\Delta\sigma$. Cette variation est obtenue de façon algébrique de différentes manières à partir de l'utilisation de l'équation (17)

$$\Delta\sigma = \left(\sigma\left(f\right)-\sigma l\right)\left(\left(\frac{fc}{f}\right)^{2}+1\right)$$

Une seule fréquence de mesure peut être utilisée si $\sigma l$ est négligeable devant $\Delta\sigma$.

Dans le cas le plus courant, il est utilisé au moins deux fréquences pour déterminer de façon algébrique $\Delta\sigma$.

On va maintenant décrire un mode pratique de détermination de la viabilité des cellules, dans le cadre du procédé de comptage selon l'invention.

Comme décrit plus haut, la mesure d'absorbance ou d'atténuation de la lumière, plus communément appelée turbidité, est corrélée avec la quantité de biomasse en suspension. Le signal optique mesuré est corrélé à la quantité de particules en suspension ou au nombre de cellules totales (vivante et morte) :

$$\text{Absorbance} = K' * Nt$$

$$\text{Avec } Nt = \text{nombre de cellules totales}$$

La détermination de l'absorbance est une mesure directe et en ligne du nombre de cellules totales après détermination de K' par une culture préalable et la corrélation avec une contre mesure du nombre de cellules totales, par exemple la méthode de référence citée ci-dessus.

La viabilité cellulaire est déterminée par l'expression suivante :

$$Nv * 100 \ / \ Nt = \text{Viabilité cellulaire}$$

Les mesures du nombre de cellules totales et nombre de cellules vivantes dans les cultures suivantes seront utilisées pour déterminer la viabilité cellulaire.

On va maintenant décrire, en référence aux figures précitées, un exemple de mise en oeuvre du procédé de comptage selon l'invention, en même temps qu'un système de comptage mettant en oeuvre ce procédé.

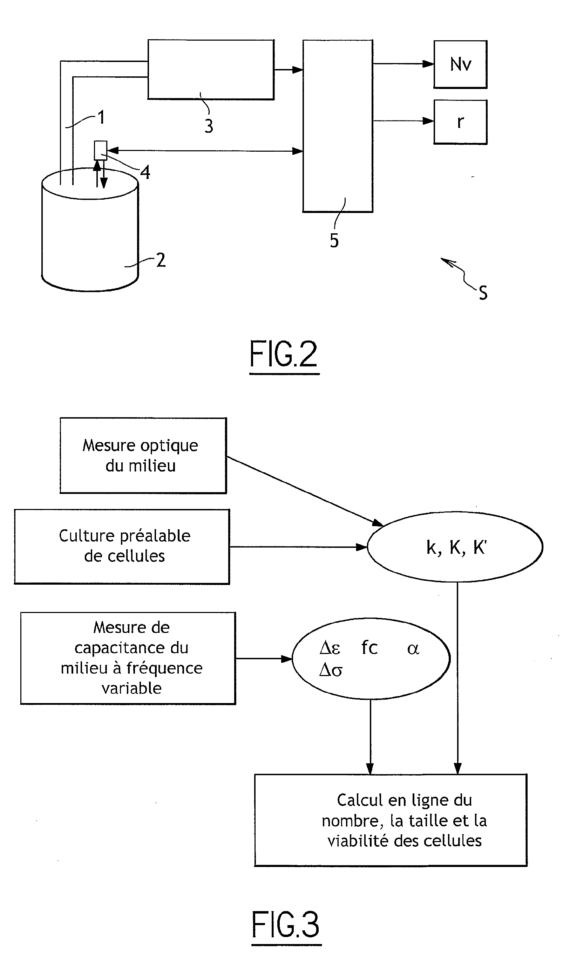

Un système S de comptage de cellules comprend, en référence à la figure 2 :

- un dispositif d'électrodes capacitives 1 plongées dans une suspension cellulaire au sein d'un fermenteur 2,
- une unité de contrôle électronique 3, comprenant par exemple un pont flottant et prévue pour générer une fréquence d'excitation variable et pour générer un signal de capacitance,
- une unité de traitement 5 pour extraire de ce signal de capacitance variant en fonction de la fréquence, des informations relatives à la $\beta$-dispersion du milieu, notamment la fréquence critique fc, la variation de permittivité $\Delta\varepsilon$, et pour délivrer une mesure en ligne du nombre de cellules vivantes et éventuellement du rayon moyen de ces cellules,
- une cellule optique 4 de mesure de l'absorbance du milieu.

En référence à la figure 3, le procédé de comptage selon l'invention est implémenté en pratique sous la forme d'un logiciel de traitement de grandeurs physiques telles que la capacitance et l'absorbance. Ce logiciel est exécuté pour traiter ces mesures d'absorbance et de capacitance variant en fonction de la fréquence pour générer les paramètres caractéristiques de la $\beta$-dispersion dans le milieu.

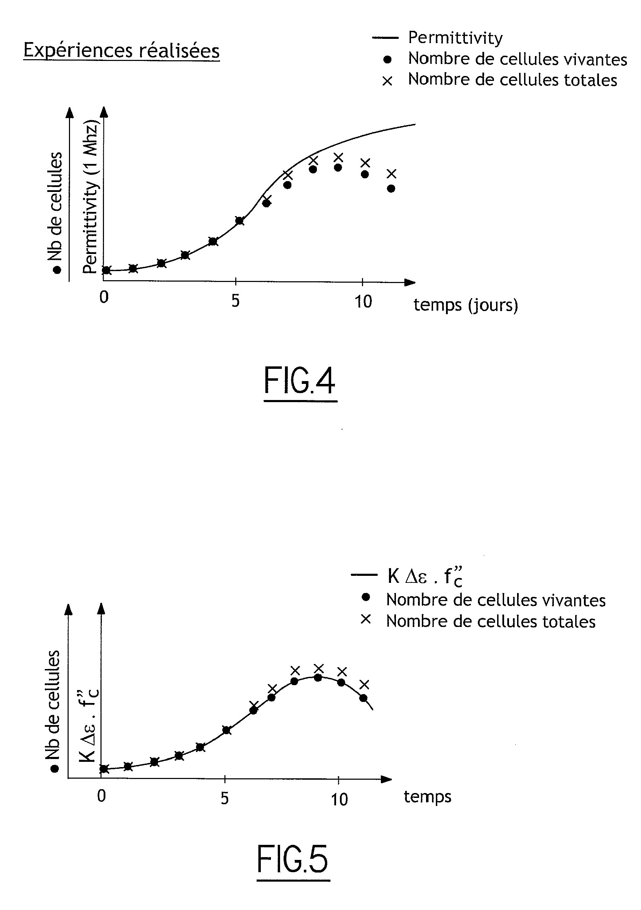

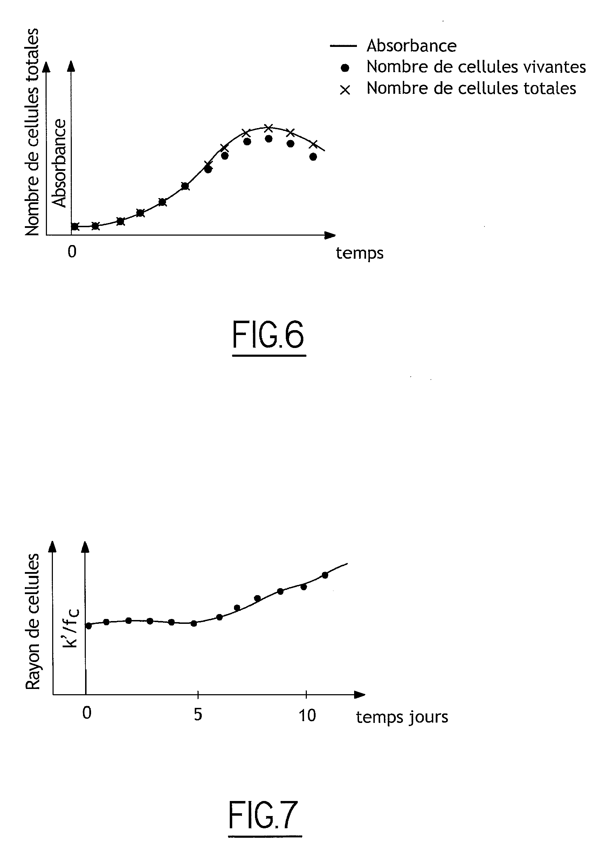

Des résultats expérimentaux sont illustrés sur les figures 4 à 7.

Bien sûr, l'invention n'est pas limitée aux exemples qui viennent d'être décrits et de nombreux aménagements peuvent être apportés à ces exemples sans sortir du cadre de l'invention.

Les signaux de capacitance et (ou) de conductance peuvent ainsi provenir de tout type d'appareil de mesure d'impédance (impédancemétrie) équipé d'un balayage en fréquence et relié à un capteur plongé dans un milieu biologique.

Sous le terme d'impédancemétrie, sont comprises :

- les différentes méthodes de mesures d'impédance basées sur la détermination du ratio V/I d'un échantillon à mesurer dont certaines sont décrites dans l'ouvrage « Impédance Measurement Handbook » publié par la compagnie « Hewlett Packard »,
- les méthodes de zéro telles que celle divulguée dans le document WO 0179828 au nom du présent déposant,
- la méthode de résonance, et plus généralement,
- toute méthode permettant de déterminer la conductance et la capacitance du milieu biologique dans lequel est plongé le capteur.

Références bibliographiques

[1] John E. Yardley, Robert Todd, David J. Nicholson, John Barrett, Douglas B. Kell, Christopher L. Davey, Correction of the influence of baseline artefacts and electrode polarisation on dielectric spectra, Bioelectrochemistry 51 (2000) 53-65.

[2] David J. Nicholson, Douglas B. Kell, Christopher L. Davey, Deconvolution of the dielectric spectra of microbial cell suspensions using multivariate calibration and artificial neural networks, Bioelectrochemistry and Bioenergetics 39 (1996) 185-193.

[3] C. L. Davey, H. M. Davey and D. B. Kell (1992) On the dielectric properties of cell suspensions at high volume fractions. Bioelectrochemistry and Bioenergetics 28 pp:319-330.

[4] Christopher L. Davey, Gerard H. Mark and Douglas B. Kell. (1993) . On the dielectric method of monitoring cellular Viability. Pure &App/. Chern., Vol. 65, No. 9, pp. 1921-1926,1993.

[5] Steffen Archer, Hywel Morgan, and Frazer J. Rixon, Electrorotation Studies of Baby Hamster Kidney Fibroblasts Infected with Herpes Simplex Virus Type 1, Biophysical Journal Volume 76 May 1999 2833-2842.

[6] Christopher Cannizzaro, Raphael Gu ¨ gerli, Ian Marison, Urs von Stockar, On-Line Biomass Monitoring of CHO Perfusion Culture With Scanning Dielectric Spectroscopy, 24 September 2003 in Wiley InterScience (www.interscience.wiley.com).

[7] Koji Asami, Takeshi Yonezawa, Dielectric analysis of yeast cell growth, Biochimica et Biophysica Acta 1245 (1995) 99-105.

[8] F. Bordi, C. Cametti, T. Gili, Reduction of the contribution of electrode polarization effects in the radiowave electric measurements of highly conductive biological cell suspensions. Bioelectrochemistry 54 (2001), 53-61.

**Revendications**

1. Procédé pour compter en ligne et *in situ* des cellules dans un milieu de culture biologique, comprenant les étapes suivantes:

    - une pluralité de mesures de la capacitance dudit milieu ou une pluralité de mesure de la conductance dudit milieu, à des fréquences distinctes variant dans une plage de fréquences de mesure prédéterminée,
    - une extraction d'une information de variation de permittivité due à la β-dispersion dans ledit milieu, à partir desdites mesures de capacitance, et
    - un traitement de ladite information de variation de permittivité pour délivrer une information de comptage de cellules dans ledit milieu.

2. Procédé selon la revendication 1, **caractérisé en ce qu'**il comprend en outre une étape préalable de calibration d'une culture desdites cellules, dite culture de calibration, cette étape de calibration comportant :

- (i) au moins une pluralité de mesures de permittivité de ladite culture de calibration à des fréquences prédéterminées,
- (ii) un traitement desdites mesures de permittivité pour calculer des facteurs de détermination des informations de comptage.

3. Procédé selon l'une des revendications 1 ou 2, **caractérisé en ce que** l'étape préalable de calibration comprend en outre une opération de corrélation des mesures de permittivité avec une mesure hors ligne permettant la mesure de quantité de biomasse totale de la culture de calibration utilisée comme référence.

4. Procédé selon l'une quelconque des revendications précédentes, **caractérisé en ce que** l'étape d'extraction d'une information de variation de permittivité due à la β-dispersion comprend une détermination de paramètres descriptifs de cette β-dispersion, parmi l'un au moins des trois paramètres suivants : la variation de permittivité $\Delta\varepsilon$, la variation de la conductance $\Delta\sigma$, la fréquence critique fc, et le paramètre $\alpha$.

5. Procédé selon l'une quelconque des revendications précédentes et la revendication 2, **caractérisé en ce que** l'étape préalable de calibration est prévue pour déterminer une fonction **k** correspondant au produit du rayon r des cellules du milieu et de la fréquence critique fc de la β-dispersion.

6. Procédé selon la revendication 5, **caractérisé en ce qu'**il comprend en outre une étape pour fournir une mesure en ligne du rayon moyen des cellules du milieu, à partir de la détermination de la fréquence critique fc.

7. Procédé selon la revendication 6, **caractérisé en ce qu'**il comprend en outre une détermination du nombre de cellules vivantes dans le milieu, à partir du rayon moyen d'une cellule déterminé en ligne.

8. Procédé selon la revendication 6, **caractérisé en ce qu'**il comprend en outre une étape de mesure directe hors ligne du rayon moyen et une étape de corrélation entre la mesure directe et la mesure indirecte en ligne dudit rayon moyen obtenue à partir de la détermination en ligne de la fréquence critique fc.

9. Procédé selon la revendication 8, **caractérisé en ce que** la mesure directe hors ligne du rayon moyen est réalisée par microscopie.

10. Procédé selon l'une quelconque des revendications précédentes, **caractérisé en ce qu'**il comprend en outre une détermination du nombre de cellules vivantes ou biovolume selon la formule :

$$\mathbf{Nv = K * \Delta\varepsilon * fc^4}$$

11. Procédé selon l'une quelconque des revendications précédentes, **caractérisé en ce qu'**il comprend en outre une détermination du nombre de cellules vivantes ou biovolume selon la formule :

$$\mathbf{Nv = K * \Delta\sigma * fc^3 \, / \, 2\,\pi}$$

12. Procédé selon l'une des revendications 10 ou 11 et la revendication 2, **caractérisé en ce que** l'étape préalable de calibration est agencée pour déterminer la fonction **K.**

13. Procédé selon l'une quelconque des revendications précédentes et la revendication 3, **caractérisé en ce que** la mesure optique permettant la mesure de quantité de biomasse totale dans la culture de calibration est agencée pour délivrer un signal optique, tel que

$$\text{absorbance} = \mathbf{K'} * Nt$$

Avec Nt = nombre de cellules totales

**14.** Procédé selon la revendication 13, **caractérisé en ce que** l'étape de calibration est agencée pour déterminer le coefficient **K'.**

**15.** Procédé selon la revendication 14, **caractérisé en ce qu'**il comprend en outre une mesure directe en ligne du nombre Nt de cellules totales, à partir du signal optique permettant la mesure de quantité de biomasse totale mesuré et du coefficient K' déterminé lors de l'étape de culture préalable.

**16.** Procédé selon la revendication 15, **caractérisé en ce qu'**il comprend en outre une fourniture d'une information de viabilité cellulaire selon la formule :

$$Nv *100 / Nt = \text{Viabilité cellulaire}$$

**17.** Procédé selon l'une quelconque des revendications précédentes, **caractérisé en ce que** la plage de fréquences de mesure est comprise entre 0.1 et 20 MHz.

**18.** Système pour compter en ligne et *in situ* des cellules dans un milieu de culture biologique, mettant en oeuvre le procédé selon l'une quelconque des revendications précédentes, comprenant :

- des moyens pour mesurer la capacitance dudit milieu, à des fréquences distinctes variant dans une plage de fréquences de mesure prédéterminée,
- des moyens pour extraire une information de variation de permittivité due à la β-dispersion dans ledit milieu, à partir desdites mesures de capacitance, et
- des moyens pour traiter ladite information de variation de permittivité, pour délivrer une information de comptage de cellules dans ledit milieu.

**19.** Système selon la revendication 18, **caractérisé en ce qu'**il comprend en outre des moyens pour réaliser une calibration d'une culture desdites cellules, dite culture de calibration, comportant :

- (i) des moyens pour effectuer des mesures de permittivité de ladite culture de calibration à des fréquences prédéterminées, et
- (ii) des moyens pour traiter lesdites mesures de permittivité, agencés pour calculer des facteurs de détermination des informations de comptage.

**20.** Système selon l'une des revendication 18 ou 19, **caractérisé en ce qu'**il comprend en outre des moyens pour effectuer une mesure directe hors ligne du rayon moyen des cellules du milieu, et des moyens pour réaliser une corrélation entre ladite mesure directe et la mesure indirecte en ligne dudit rayon moyen obtenue à partir de la détermination en ligne de la fréquence critique fc dudit milieu.

**21.** Système selon la revendication 20, **caractérisé en ce qu'**il comprend en outre, au titre des moyens de mesure directe hors ligne du rayon moyen, un équipement de microscopie.

FIG.1

FIG.2

| Mesure optique du milieu |
| Culture préalable de cellules |
| Mesure de capacitance du milieu à fréquence variable |

k, K, K'

$\Delta\varepsilon$   fc   $\alpha$
$\Delta\sigma$

Calcul en ligne du nombre, la taille et la viabilité des cellules

FIG.3

Expériences réalisées

— Permittivity
● Nombre de cellules vivantes
✕ Nombre de cellules totales

FIG.4

— K Δε . f"$_C$
● Nombre de cellules vivantes
✕ Nombre de cellules totales

FIG.5

Nombre de cellules totales

Absorbance

—— Absorbance
● Nombre de cellules vivantes
✕ Nombre de cellules totales

0                                                    temps

## FIG.6

Rayon de cellules

$k'/f_C$

0               5               10        temps jours

## FIG.7

Europäisches
Patentamt

European
Patent Office

Office européen
des brevets

# RAPPORT DE RECHERCHE EUROPEENNE

Numéro de la demande

EP 08 15 8182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 874 264 A (NANOTEC SOLUTION SOC CIV ILE [FR]) 17 février 2006 (2006-02-17) * revendications 1-54 * | 1-21 | INV. C12M1/34 G01N33/487 |
| A,D | BORDI F ET AL: "Reduction of the contribution of electrode polarization effects in the radiowave dielectric measurements of highly conductive biological cell suspensions" BIOELECTROCHEMISTRY, ELESEVIER, AMSTERDAM, NL, vol. 54, 31 août 2001 (2001-08-31), pages 53-61, XP002320839 ISSN: 1567-5394 * page 56 - page 57 * | 1 | |
| A | FR 2 867 278 A (UNIV MONTPELLIER II [FR]; GERVAIS DANONE SA [FR]; NANOTEC SOLUTION [FR]) 9 septembre 2005 (2005-09-09) * revendication 1 * | 1 | |
| A | GB 2 329 711 A (UNIV WALES ABERYSTWYTH THE [GB]) 31 mars 1999 (1999-03-31) * revendications 1,4 * | 1 | **DOMAINES TECHNIQUES RECHERCHES (IPC)** C12M G01N |
| A | EP 1 085 316 A (ABER INSTR LTD [GB]) 21 mars 2001 (2001-03-21) * revendication 1 * | 1 | |
| A | FR 2 867 279 A (NANOTEC SOLUTION [FR]) 9 septembre 2005 (2005-09-09) * page 16, ligne 10 - page 17, ligne 20; revendication 1 * | 1 | |

-/--

Le présent rapport a été établi pour toutes les revendications

| Lieu de la recherche | Date d'achèvement de la recherche | Examinateur |
|---|---|---|
| Berlin | 9 décembre 2008 | Clement, Jean-Paul |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant

EPO FORM 1503 03.82 (P04C02)

**Europäisches Patentamt**
**European Patent Office**
**Office européen des brevets**

# RAPPORT DE RECHERCHE EUROPEENNE

Numéro de la demande

EP 08 15 8182

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CHRISTINE M. HARRIS ETAL: "Dielectric permittivity of microbial suspensions at radio frequencies: a novel method for the real-time estimation of microbial biomass" ENZYME MICROBIOLOGICAL TECHNOLOGY, vol. 9, mars 1987 (1987-03), pages 181-186, XP002459329 * page 182 - page 183 * ----- | | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Berlin | 9 décembre 2008 | Clement, Jean-Paul |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 08 15 8182

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-12-2008

| Document brevet cité au rapport de recherche | | Date de publication | Membre(s) de la famille de brevet(s) | | Date de publication |
|---|---|---|---|---|---|
| FR 2874264 | A | 17-02-2006 | EP | 1784480 A1 | 16-05-2007 |
| | | | WO | 2006021691 A1 | 02-03-2006 |
| | | | US | 2008262748 A1 | 23-10-2008 |
| FR 2867278 | A | 09-09-2005 | EP | 1730505 A2 | 13-12-2006 |
| | | | WO | 2005085818 A2 | 15-09-2005 |
| GB 2329711 | A | 31-03-1999 | DE | 69812394 D1 | 24-04-2003 |
| | | | DE | 69812394 T2 | 19-02-2004 |
| | | | EP | 1018025 A1 | 12-07-2000 |
| | | | WO | 9917124 A1 | 08-04-1999 |
| | | | US | 6496020 B1 | 17-12-2002 |
| EP 1085316 | A | 21-03-2001 | GB | 2354588 A | 28-03-2001 |
| FR 2867279 | A | 09-09-2005 | WO | 2005085412 A2 | 15-09-2005 |

EPO FORM P0460

Pour tout renseignement concernant cette annexe : voir Journal Officiel de l'Office européen des brevets, No.12/82

**EP 2 025 744 A1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0179828 A

**Littérature non-brevet citée dans la description**

- **JOHN E. YARDLEY ; ROBERT TODD ; DAVID J. NICHOLSON ; JOHN BARRETT ; DOUGLAS B. KELL ; CHRISTOPHER L. DAVEY.** Correction of the influence of baseline artefacts and electrode polarisation on dielectric spectra. *Bioelectrochemistry,* 2000, vol. 51, 53-65
- **DAVID J. NICHOLSON ; DOUGLAS B. KELL ; CHRISTOPHER L. DAVEY.** Deconvolution of the dielectric spectra of microbial cell suspensions using multivariate calibration and artificial neural networks. *Bioelectrochemistry and Bioenergetics,* 1996, vol. 39, 185-193
- **C. L. DAVEY ; H. M. DAVEY ; D. B. KELL.** On the dielectric properties of cell suspensions at high volume fractions. *Bioelectrochemistry and Bioenergetics,* 1992, vol. 28, 319-330
- **CHRISTOPHER L. DAVEY ; GERARD H. MARK ; DOUGLAS B. KELL.** On the dielectric method of monitoring cellular Viability. *Pure &App/. Chern.,* 1993, vol. 65 (9), 1921-19261993
- **STEFFEN ARCHER ; HYWEL MORGAN ; FRAZER J. RIXON.** Electrorotation Studies of Baby Hamster Kidney Fibroblasts Infected with Herpes Simplex Virus Type 1. *Biophysical Journal,* Mai 1999, vol. 76, 2833-2842
- **CHRISTOPHER CANNIZZARO ; RAPHAEL GU ̈ GERLI ; IAN MARISON ; URS VON STOCKAR.** On-Line Biomass Monitoring of CHO Perfusion Culture With Scanning Dielectric Spectroscopy. Wiley InterScience, 24 Septembre 2003
- **KOJI ASAMI ; TAKESHI YONEZAWA.** Dielectric analysis of yeast cell growth. *Biochimica et Biophysica Acta,* 1995, vol. 1245, 99-105
- **F. BORDI ; C. CAMETTI ; T. GILI.** Reduction of the contribution of electrode polarization effects in the radiowave electric measurements of highly conductive biological cell suspensions. *Bioelectrochemistry,* 2001, vol. 54, 53-61